# EUROPEAN PATENT SPECIFICATION

(11) **EP 3 422 881 B1**
(45) Date of publication and mention of the grant of the patent: **16.07.2025**
(21) Application number: 17711341.2
(22) Date of filing: 01.03.2017
(51) Int. Cl.: A24F 47/00

(54) **FLEXIBLE DISPLAY FOR AN AEROSOL DELIVERY DEVICE**
FLEXIBLE ANZEIGE FÜR EINE AEROSOLABGABEVORRICHTUNG
AFFICHAGE FLEXIBLE POUR UN DISPOSITIF D'ADMINISTRATION D'AÉROSOL

(30) Priority: 04.03.2016 US 201615061529
(43) Date of publication of application: 09.01.2019
(73) Proprietor: RAI Strategic Holdings, Inc., Winston-Salem, NC 27101 (US)
(72) Inventor: LAMB, Wilson Christopher, Hillsborough, NC 27278 (US); AMPOLINI, Frederic Philippe, Winston-Salem, NC 27106 (US); HENRY, JR., Raymond C., Green Cove Springs, FL 32043 (US)
(74) Representative: Hoeger, Stellrecht & Partner Patentanwälte mbB
(86) International application number: PCT/IB2017/051207
(87) International publication number: WO 2017/149481

(56) References cited:
- CN-U- 203 505 584
- US-A1- 2013 104 916
- US-A1- 2015 122 252
- US-A1- 2015 257 445
- US-A1- 2015 272 220
- US-A1- 2015 272 222

## Description

### TECHNOLOGICAL FIELD

The present disclosure relates to aerosol delivery devices such as smoking articles, and more particularly to aerosol delivery devices that may utilize electrically generated heat for the production of aerosol (e.g., smoking articles commonly referred to as electronic cigarettes). The smoking articles may be configured to heat an aerosol precursor, which may incorporate materials that may be made or derived from, or otherwise incorporate tobacco, the precursor being capable of forming an inhalable substance for human consumption.

### BACKGROUND

US 2015/122252 A1 discloses a hand-held personal vaporizer including a cylindrical housing, a vaporizer material disposed within the cylindrical housing and a vaporizer control module functionally coupled to the vaporizer and configured to control function of the vaporizer.

US 2015/272220 A1 discloses an electronic nicotine delivery device including a nicotine dosage sensor that determines an amount of nicotine consumed by a user based on the duration of inhalation.

US 2015/272222 A1 discloses an inhalation sensor for an alternative nicotine or THC delivery device having a diluted nicotine or THC solution. The inhalation sensor includes a processor and memory. The memory includes a solution profile including information indicative of the nicotine or THC concentration of the diluted nicotine or THC solution and a device profile including information indicative of the alternative nicotine or THC delivery device.

CN 203505584 U discloses an electronic cigarette with an E-ink electronic paper display. The electronic E-ink display screen is arranged on an outer shell.

US 2015/257445 A1 discloses a method including an aerosol delivery device determining a characteristic of a user input to the aerosol delivery device.

Many smoking devices have been proposed through the years as improvements upon, or alternatives to, smoking products that require combusting tobacco for use. Many of those devices purportedly have been designed to provide the sensations associated with cigarette, cigar or pipe smoking, but without delivering considerable quantities of incomplete combustion and pyrolysis products that result from the burning of tobacco. To this end, there have been proposed numerous smoking products, flavor generators and medicinal inhalers that utilize electrical energy to vaporize or heat a volatile material, or attempt to provide the sensations of cigarette, cigar or pipe smoking without burning tobacco to a significant degree. See, for example, the various alternative smoking articles, aerosol delivery devices and heat generating sources set forth in the background art described in U.S. Pat. Nos. 7,726,320 to Robinson et al. and 8,881,737 to Collett et al. See also, for example, the various types of smoking articles, aerosol delivery devices and electrically-powered heat generating sources referenced by brand name and commercial source in U.S. Pat. Pub. No. 2015/0216232 to Bless et al.. Additionally, various types of electrically powered aerosol and vapor delivery devices also have been proposed in U.S. Pat. Pub. Nos. 2014/0096781 to Sears et al. and 2014/0283859 to Minskoff et al., as well as U.S. Pat. App. Ser. Nos. 14/282,768 to Sears et al., filed May 20, 2014; 14/286,552 to Brinkley et al., filed May 23, 2014; 14/327,776 to Ampolini et al., filed July 10, 2014; and 14/465,167 to Worm et al., filed August 21, 2014.

### BRIEF SUMMARY

The present disclosure relates to aerosol delivery devices, methods of forming such devices, and elements of such devices. In accordance with example implementations of the present disclosure, an aerosol delivery device is equipped with a flexible emissive display capable of present information such as an approximate remaining capacity of the device's power supply or aerosol precursor composition (e.g., in 10-20% increments), which may provide a user sufficient feedback to anticipate the need to charge or replace the power supply or cartridge. The emissive display is a flexible solution that does not include glass or other fragile components, and may be more easily contoured to various device form factors. The emissive display may be low-cost and is moldable to various form factors of aerosol delivery devices. And the emissive display may be included on an aerosol delivery device or various of its components, such as its power supply or cartridge.

The present disclosure thus includes, without limitation, the following:
An apparatus comprising at least one housing equipped with a heating element and containing an aerosol precursor composition, the heating element being configured to activate and vaporize components of the aerosol precursor composition; and a flexible emissive display received on a surface of the at least one housing, wherein the flexible, emissive display is flexible so as to be moldable to a form factor of the surface of the at least one housing, the surface being non-planar, and controllable to present information of the aerosol delivery device, wherein the apparatus is an aerosol delivery device or a cartridge for an aerosol delivery device.

The apparatus of above, wherein the non-planar surface is substantially tubular in shape.

The apparatus of above, wherein the emissive display is controllable to present information that indicates an amount of aerosol precursor composition contained in or consumed from the at least one housing.

The apparatus of above, wherein the emissive display being controllable to present information includes being controllable to present an icon that represents the aerosol precursor composition and indicates the amount thereof contained in or consumed from the at least one housing.

The apparatus of above, wherein the heating element is connected or connectable with a power source, and the emissive display is controllable to present information that indicates an amount of charge contained in or consumed from the power source.

The apparatus of above, wherein the emissive display being controllable to present information includes being controllable to present an icon that represents the power source and indicates the amount of charge contained in or consumed therefrom.

The apparatus of above, wherein the emissive display being controllable to present information includes being controllable to present information that also indicates an amount of aerosol precursor composition contained in or consumed from the at least one housing.

The apparatus of above, wherein the emissive display being controllable to present information includes being controllable to present an icon that represents the power source and indicates the amount of charge contained in or consumed therefrom, and an icon that represents the aerosol precursor composition and indicates the amount thereof contained in or consumed from the at least one housing.

A control body coupled or coupleable with a cartridge that is equipped with a heating element and contains an aerosol precursor composition, the control body being coupled or coupleable with the cartridge to form an aerosol delivery device in which the heating element is configured to activate and vaporize components of the aerosol precursor composition, the control body comprising at least one housing; a flexible emissive display received on a surface of the at least one housing; and a control component contained within the at least one housing and configured to operate in an active mode in which the control body is coupled with the cartridge, the control component in the active mode being configured to control the heating element to activate and vaporize components of the aerosol precursor composition, the control component being further configured to control the emissive display to present information of the cartridge, control body or aerosol delivery device, wherein the flexible, emissive display is flexible so as to be moldable to a form factor of the surface of the at least one housing if the surface is non-planar.

The control body of above, wherein the non-planar surface is substantially tubular in shape.

The control body of above, wherein the control component is configured to control the emissive display to present information that indicates an amount of aerosol precursor composition contained in or consumed from the at least one housing.

The control body of above, wherein the control component being configured to control the emissive display includes being configured to control the emissive or electronic paper display to present an icon that represents the aerosol precursor composition and indicates the amount thereof contained in or consumed from the at least one housing.

The control body of above, wherein the heating element is connected or connectable with a power source, and the control component is configured to control the emissive display to present information that indicates an amount of charge contained in or consumed from the power source.

The control body of above, wherein the control component being configured to control the emissive display includes being configured to control the emissive or electronic paper display to present an icon that represents the power source and indicates the amount of charge contained in or consumed therefrom.

The control body of above, wherein the control component being configured to control the emissive display includes being configured to control the emissive display to present information that also indicates an amount of aerosol precursor composition contained in or consumed from the at least one housing.

The control body of above, wherein the control component being configured to control the emissive display includes being configured to control the emissive display to present an icon that represents the power source and indicates the amount of charge contained in or consumed therefrom, and an icon that represents the aerosol precursor composition and indicates the amount thereof contained in or consumed from the at least one housing.

These and other features, aspects, and advantages of the present disclosure will be apparent from a reading of the following detailed description together with the accompanying drawings, which are briefly described below. This disclosure is intended to be read holistically such that any separable features or elements of the disclosure, in any of its aspects and example implementations, should be viewed as combinable, unless the context of the disclosure clearly dictates otherwise.

It will therefore be appreciated that this Brief Summary is provided merely for purposes of summarizing some example implementations so as to provide a basic understanding of some aspects of the disclosure. Accordingly, it will be appreciated that the above described example implementations are merely examples and should not be construed to narrow the scope of the disclosure in any way Other example implementations, aspects and advantages will become apparent from the following detailed description taken in conjunction with the accompanying drawings which illustrate, by way of example, the principles of some described example implementations.

### BRIEF DESCRIPTION OF THE FIGURES

Having thus described the disclosure in the foregoing general terms, reference will now be made to the accompanying drawings, which are not necessarily drawn to scale, and wherein:
FIG. 1 illustrates a perspective view of an aerosol delivery device including a cartridge, a control body and a flexible, emissive or electronic paper display, according to an example implementation of the present disclosure;
FIGS. 2 and 3 illustrate exploded views of respectively the cartridge and control body of FIG. 1, according to an example implementation of the present disclosure; and
FIG. 4 illustrates information that may be presented by the emissive or electronic paper display of FIG. 1, according to an example implementation of the present disclosure.

### DETAILED DESCRIPTION

The present disclosure will now be described more fully hereinafter with reference to example implementations thereof. These example implementations are described so that this disclosure will be thorough and complete, and will fully convey the scope of the disclosure to those skilled in the art. Indeed, the disclosure may be embodied in many different forms and should not be construed as limited to the implementations set forth herein; rather, these implementations are provided so that this disclosure will satisfy applicable legal requirements. As used in the specification and the appended claims, the singular forms "a," "an," "the" and the like include plural referents unless the context clearly dictates otherwise.

As described hereinafter, example implementations of the present disclosure relate to aerosol delivery systems. Aerosol delivery systems according to the present disclosure use electrical energy to heat a material (preferably without combusting the material to any significant degree) to form an inhalable substance; and components of such systems have the form of articles most preferably are sufficiently compact to be considered hand-held devices. That is, use of components of preferred aerosol delivery systems does not result in the production of smoke in the sense that aerosol results principally from byproducts of combustion or pyrolysis of tobacco, but rather, use of those preferred systems results in the production of vapors resulting from volatilization or vaporization of certain components incorporated therein. In some example implementations, components of aerosol delivery systems may be characterized as electronic cigarettes, and those electronic cigarettes most preferably incorporate tobacco and/or components derived from tobacco, and hence deliver tobacco derived components in aerosol form.

Aerosol generating pieces of certain preferred aerosol delivery systems may provide many of the sensations (e.g., inhalation and exhalation rituals, types of tastes or flavors, organoleptic effects, physical feel, use rituals, visual cues such as those provided by visible aerosol, and the like) of smoking a cigarette, cigar or pipe that is employed by lighting and burning tobacco (and hence inhaling tobacco smoke), without any substantial degree of combustion of any component thereof. For example, the user of an aerosol generating piece of the present disclosure can hold and use that piece much like a smoker employs a traditional type of smoking article, draw on one end of that piece for inhalation of aerosol produced by that piece, take or draw puffs at selected intervals of time, and the like.

Aerosol delivery systems of the present disclosure also can be characterized as being vapor-producing articles or medicament delivery articles. Thus, such articles or devices can be adapted so as to provide one or more substances (e.g., flavors and/or pharmaceutical active ingredients) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases and aerosols of a form or type suitable for human inhalation, whether or not visible, and whether or not of a form that might be considered to be smoke-like.

Aerosol delivery systems of the present disclosure generally include a number of components provided within an outer body or shell, which may be referred to as a housing. The overall design of the outer body or shell can vary, and the format or configuration of the outer body that can define the overall size and shape of the aerosol delivery device can vary. Typically, an elongated body resembling the shape of a cigarette or cigar can be a formed from a single, unitary housing or the elongated housing can be formed of two or more separable bodies. For example, an aerosol delivery device can comprise an elongated shell or body that can be substantially tubular in shape and, as such, resemble the shape of a conventional cigarette or cigar. In one example, all of the components of the aerosol delivery device are contained within one housing. Alternatively, an aerosol delivery device can comprise two or more housings that are joined and are separable. For example, an aerosol delivery device can possess at one end a control body comprising a housing containing one or more reusable components (e.g., an accumulator such as a rechargeable battery, thin film solid state battery and/or capacitor, and various electronics for controlling the operation of that article), and at the other end and removably coupleable thereto, an outer body or shell containing a disposable portion (e.g., a disposable flavor-containing cartridge).

Aerosol delivery systems of the present disclosure most preferably comprise some combination of a power source (i.e., an electrical power source), at least one control component (e.g., means for actuating, controlling, regulating and ceasing power for heat generation, such as by controlling electrical current flow the power source to other components of the article - e.g., a microprocessor, individually or as part of a microcontroller), a heater or heat generation member (e.g., an electrical resistance heating element or other component, which alone or in combination with one or more further elements may be commonly referred to as an "atomizer"), an aerosol precursor composition (e.g., commonly a liquid capable of yielding an aerosol upon application of sufficient heat, such as ingredients commonly referred to as "smoke juice," "e-liquid" and "e-juice"), and a mouthend region or tip for allowing draw upon the aerosol delivery device for aerosol inhalation (e.g., a defined airflow path through the article such that aerosol generated can be withdrawn therefrom upon draw).

More specific formats, configurations and arrangements of components within the aerosol delivery systems of the present disclosure will be evident in light of the further disclosure provided hereinafter. Additionally, the selection and arrangement of various aerosol delivery system components can be appreciated upon consideration of the commercially available electronic aerosol delivery devices, such as those representative products referenced in background art section of the present disclosure.

In various examples, an aerosol delivery device can comprise a reservoir configured to retain the aerosol precursor composition. The reservoir particularly can be formed of a porous material (e.g., a fibrous material) and thus may be referred to as a porous substrate (e.g., a fibrous substrate).

A fibrous substrate useful as a reservoir in an aerosol delivery device can be a woven or nonwoven material formed of a plurality of fibers or filaments and can be formed of one or both of natural fibers and synthetic fibers. For example, a fibrous substrate may comprise a fiberglass material. In particular examples, a cellulose acetate material can be used. In other example implementations, a carbon material can be used. A reservoir may be substantially in the form of a container and may include a fibrous material included therein.

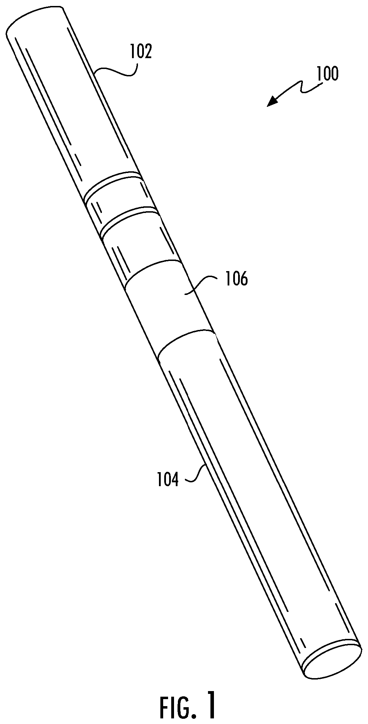

FIG. 1 illustrates a perspective view of an aerosol delivery device **100** including a cartridge **102** and a control body **104,** according to various example implementations of the present disclosure. In particular, FIG. 1 illustrates the control body and the cartridge coupled to one another. The control body and the cartridge may be detachably aligned in a functioning relationship. Various mechanisms may connect the cartridge to the control body to result in a threaded engagement, a press-fit engagement, an interference fit, a magnetic engagement or the like.

The aerosol delivery device **100** may be substantially rod-like or tubular (or cylindrically) shaped in some example implementations when the cartridge **102** and the control body **104** are in an assembled configuration. In other examples, further shapes and dimensions are encompassed - e.g., a rectangular or triangular cross-section, multifaceted shapes, or the like. The aerosol delivery device may also be substantially rectangular or rhomboidal in cross-section, which may lend itself to greater compatibility with a substantially flat or thin-film power source, such as a power source including a flat battery. The cartridge and control body may include separate, respective housings or outer bodies, which may be formed of any of a number of different materials. The housing may be formed of any suitable, structurally-sound material. In some examples, the housing may be formed of a metal or alloy, such as stainless steel, aluminum or the like. Other suitable materials include various plastics (e.g., polycarbonate), metal-plating over plastic, ceramics and the like.

In some example implementations, one or both of the control body **104** or the cartridge **102** of the aerosol delivery device **100** may be referred to as being disposable or as being reusable. For example, the control body may have a replaceable battery or a rechargeable battery and thus may be combined with any type of recharging technology, including connection to a typical alternating current electrical outlet, connection to a car charger (i.e., a cigarette lighter receptacle), connection to a computer, such as through a universal serial bus (USB) cable or connector, or connection to a photovoltaic cell (sometimes referred to as a solar cell) or solar panel of solar cells. Further, in some example implementations, the cartridge may comprise a single-use cartridge, as disclosed in U.S. Pat. No. 8,910,639 to Chang et al.
In some example implementations, the aerosol delivery device **100** may include a flexible, emissive or electronic paper display **106** on and contoured to the housing of either or both the cartridge **102** or control body **104** (shown), whereas only the examples using a flexible emissive display are part of the claimed invention, and controllable to present information of the aerosol delivery device. In some examples, a touch-sensitive surface may overlay the emissive or electronic paper display to form a touchscreen. In these examples, the touchscreen may be configured to both present information of the aerosol delivery device, and receive input to the aerosol delivery device. According to the invention, the emissive or electronic paper display **106** is contoured to a non-planar surface of the housing of the cartridge **104** or control body **104,** such as a surface that is substantially tubular in shape. In the emissive or electronic paper display may be controllable by the control component to present information of the control body, cartridge or aerosol delivery device **100.** Examples of suitable emissive displays include those based on technologies such as amorphous silicon (a-Si), low-temperature a-Si, low-temperature polycrystalline silicon (LTPS), organic semiconductor processes, jet-printed electronics and the like. More specific examples of suitable emissive displays include a-Si thin-film transistor (TFT) displays, LTPS organic light emitting diode (OLED) displays, active matrix OLED (AMOLED) displays, and the like. Examples of suitable electronic paper displays include electronic paper or e-paper displays such as Gyricon displays, electrophoretic displays, microencapsulated electrophoretic displays, electro-wetting displays, electrofluidic displays, interferometric modulator displays, other bistable displays and the like.

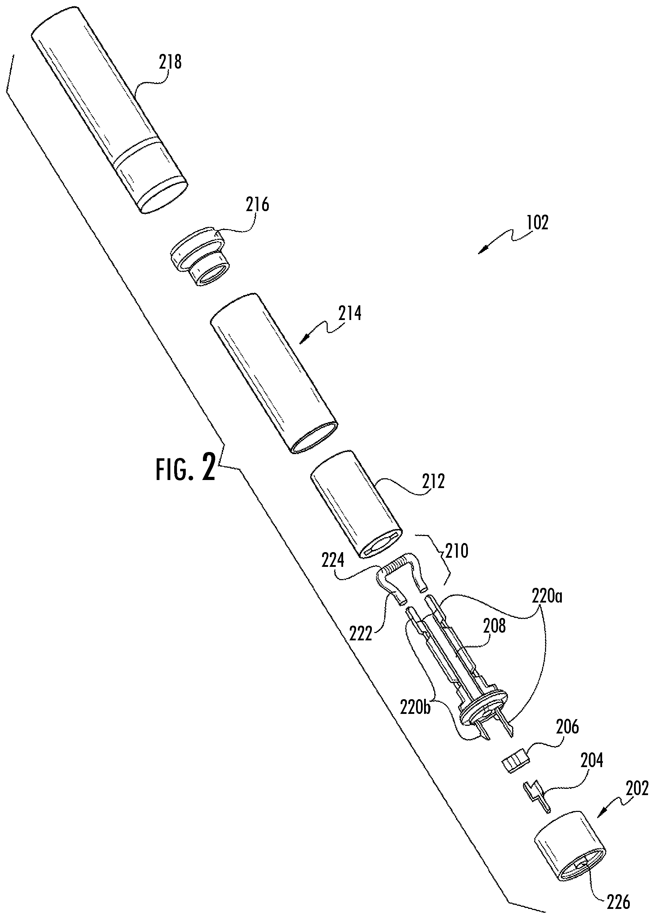

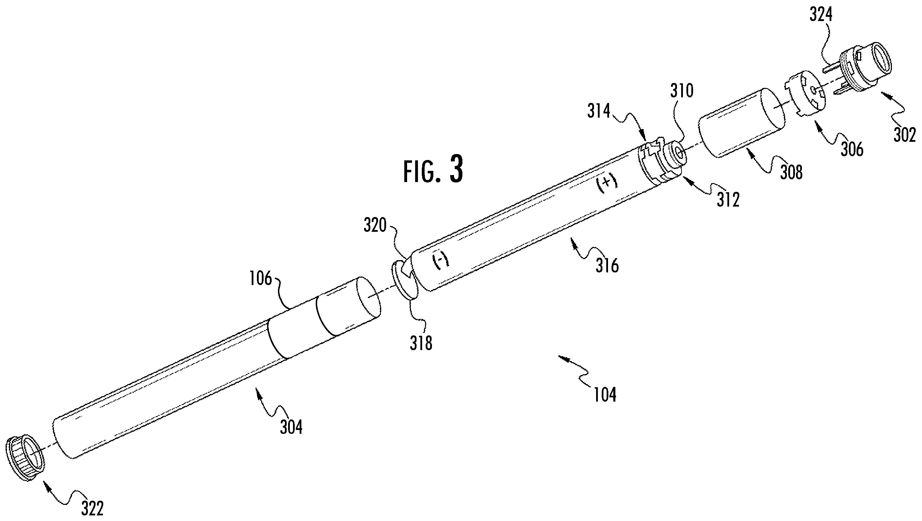

FIGS. 2 and 3 illustrate exploded views of respectively the cartridge **102** and control body **104** of FIG. 1, according to an example implementation of the present disclosure in which the control body may include an emissive or electronic paper display **106.** The components illustrated in FIGS. 2 and 3 are representative of the components that may be present in a cartridge and control body and are not intended to limit the scope of components that are encompassed by the present disclosure. It should also be understood that in other example implementations, the cartridge may include an emissive or electronic paper display in addition to or in lieu of the control body.

As illustrated in FIG. 2, the cartridge **102** may include a base **202,** a control component terminal **204,** a (electronic) control component **206,** a flow director **208,** an atomizer **210,** a reservoir substrate **212,** an outer body **214,** a mouthpiece **216,** a label **218,** and first and second heating terminals **220a, 220b.** The atomizer may include a liquid transport element **222** and a heating element **224** (sometimes referred to more simply as a heater). In some examples, the cartridge may additionally include a base shipping plug engaged with the base and/or a mouthpiece shipping plug engaged with the mouthpiece in order to protect the base and the mouthpiece and prevent entry of contaminants therein prior to use as disclosed, for example, in U.S. Pat. App. Pub. No. 2014/0261408 to Depiano et al. In various configurations, this structure may be referred to as a tank; and accordingly, the terms "cartridge," "tank" and the like may be used interchangeably to refer to an outer body or other housing enclosing a reservoir (reservoir substrate) for aerosol precursor composition, and including a heating element.

The base **202** and mouthpiece **216** may be coupled to respectively first and second opposing ends of the outer body **214** to enclose the remaining components of the cartridge **102** therein. The base may be configured to engage the control body **104.** In some examples, the base may include anti-rotation features that substantially prevent relative rotation between the cartridge and the control body as disclosed in U.S. Pat. App. Pub. No. 2014/0261495 to Novak et al. The label **218** may at least partially surround one or more of the outer body, base or mouthpiece, and include information such as a product identifier thereon.

Various components may be received within the outer body **214** and positioned between the base **202** and the mouthpiece **216.** For example, the control component terminal **204,** the control component **206**, the flow director **208,** the atomizer **210,** and the reservoir substrate **212** may be retained within the outer body. The atomizer may include a first heating terminal **220a** and a second heating terminal **220b,** a liquid transport element **222** and a heating element **224.** In this regard, the reservoir substrate may be configured to hold an aerosol precursor composition, which is directed to the heating element via the liquid transport element, as described below.

The aerosol precursor composition, also referred to as a vapor precursor composition, may comprise a variety of components including, by way of example, a polyhydric alcohol (e.g., glycerin, propylene glycol or a mixture thereof), nicotine, tobacco, tobacco extract and/or flavorants. Representative types of aerosol precursor components and formulations also are set forth and characterized in U.S. Pat. No. 7,217,320 to Robinson et al. and U.S. Pat. Pub. Nos. 2013/0008457 to Zheng et al.; 2013/0213417 to Chong et al.; 2014/0060554 to Collett et al.; 2015/0020823 to Lipowicz et al.; and 2015/0020830 to Koller, as well as WO 2014/182736 to Bowen et al. Other aerosol precursors that may be employed include the aerosol precursors that have been incorporated in the VUSE^{®} product by R. J. Reynolds Vapor Company, the BLU^{™} product by Imperial Tobacco Group PLC, the MISTIC MENTHOL product by Mistic Ecigs, and the VYPE product by CN Creative Ltd. Also desirable are the so-called "smoke juices" for electronic cigarettes that have been available from Johnson Creek Enterprises LLC.

The reservoir substrate **212** may include a plurality of layers of nonwoven fibers formed into the shape of a tube encircling the interior of the outer body **214** of the cartridge **102.** Liquid components, for example, can be sorptively retained by the reservoir substrate. The reservoir substrate is in fluid connection with the liquid transport element **222.** Thus, the liquid transport element may be configured to transport liquid from the reservoir substrate to the heating element **224** (e.g., via capillary action). Representative types of substrates, reservoirs or other components for supporting the aerosol precursor composition are described in U.S. Pat. Nos. 8,528,569 to Newton and 8,715,070 to Davis et al.; U.S. Pat. App. Pub. No. 2014/0261487 to Chapman et al.; and U.S. Pat. App. Ser. No. 14/170,838 to Bless et al., filed February 3, 2014.

As illustrated, the liquid transport element **222** may be configured to be in direct contact with the heating element **224.** Various wicking materials, and the configuration and operation of those wicking materials within certain types of aerosol delivery devices, are set forth in U.S. Pat. No. 8,910,640 to Sears et al. A variety of the materials disclosed by the
foregoing documents may be incorporated into the present devices in various example implementations, and all of the foregoing disclosures are incorporated herein by reference in their entireties.

The heating element **224** may include a wire defining a plurality of coils wound about the liquid transport element **222.** In some examples, the heating element may be formed by winding the wire about the liquid transport element as described in U.S. Pat. App. Pub. No. 2014/0157583 to Ward et al. Further, in some examples, the wire may define a variable coil spacing, as described in U.S. Pat. App. Pub. No. 2014/0270730 to DePiano et al. Various materials configured to produce heat when electrical current is applied therethrough may be employed to form the heating element. Example materials from which the wire coil may be formed include Kanthal (FeCrAl), Nichrome, Molybdenum disilicide (MoSi₂), molybdenum silicide (MoSi), Molybdenum disilicide doped with Aluminum (Mo(Si,Al)₂), graphite and graphite-based materials; and ceramic (e.g., a positive or negative temperature coefficient ceramic).

The first heating terminal **220a** and the second heating terminal **220b** (e.g., positive and negative terminals) at the opposing ends of the heating element **224** are configured to form an electrical connection with the control body **104** when the cartridge **102** is connected thereto. Further, when the control body is coupled to the cartridge, the control component **206** may form an electrical connection with the control body through the control component terminal. The control body may thus employ the control component to determine whether the cartridge is genuine and/or perform other functions. Examples of a suitable control component include one or more of each of a number of electronic components such as a microprocessor (individually or as part of a microcontroller), application-specific integrated circuit (ASIC), field-programmable gate array (FPGA) or the like. Further, various examples of control components and functions performed thereby are described in U.S. Pat. App. Pub. No. 2014/0096781 to Sears et al.

Various other details with respect to the cartridge 102 are described above are provided in U.S. Pat. App. Ser. No. 14/286,552 to Brinkley et al., filed May 23, 2014. Further, it should be understood that the cartridge may be assembled in a variety of manners and may include additional or fewer components which may be the same or different in other example implementations. For example, although the cartridge is generally described herein as including a reservoir substrate, in other examples, the cartridge may hold an aerosol precursor composition therein without the use of a reservoir substrate (e.g., through use of a container or vessel that stores the aerosol precursor composition or direct storage therein). In some examples, an aerosol precursor composition may be within a container or vessel that may also include a porous (e.g., fibrous) material therein. Further, in other examples, the aerosol precursor composition may be delivered to the atomizer via other mechanisms such as positive displacement mechanisms as disclosed in U.S. Pat. App. Ser. No. 14/309,282, filed June 19, 2014, bubble jet heads as disclosed in U.S. Pat. App. Ser. No. 14/524,778, filed October 29, 2014, and pressurized dispensers as disclosed in U.S. Pat. App. Ser. No. 14/289,101, filed May 28, 2014, each to Brammer et al. Additionally, although usage of a coil heating element is generally discussed herein, in other
examples, the atomizer may include a microheater, one or more vaporization heating elements, and/or various atomizers as disclosed, for example, in U.S. Pat. App. Ser. No. 14/309,282, filed June 19, 2014; U.S. Pat. App. Ser. No. 14/524,778, filed October 29, 2014; and U.S. Pat. App. Ser. No. Appl. No. 14/289,101, filed May 28, 2014, each to Brammer et al. and U.S. No. 8,881,737 to Collett et al.

Various components of an aerosol delivery device according to the present disclosure can be chosen from components described in the art and commercially available. Reference is made for example to the reservoir and heater system for controllable delivery of multiple aerosolizable materials in an electronic smoking article disclosed in U.S. Pat. App. Pub. No. 2014/0000638 to Sebastian et al.

Note further that portions of the cartridge **102** illustrated in FIG. 2 are optional. In this regard, by way of example, the cartridge may not include the flow director **208,** the control component terminal **204,** and/or the control component **206** in some example implementations.

In another example, substantially the entirety of the cartridge **102** may be formed from one or more carbon materials, which may provide advantages in terms of biodegradability and absence of wires. In this regard, the heating element may include carbon foam, the reservoir may include carbonized fabric, and graphite may be employed to form an electrical connection with the battery and controller. An example of a suitable carbon-based cartridge is provided in U.S. Pat. App. Pub. No. 2013/0255702 to Griffith et al.

FIG. 3 illustrates an exploded view of the control body **104** of the aerosol delivery device **100** according to an example implementation of the present disclosure. As illustrated, in addition to the emissive or electronic paper display **106,** the control body may include a coupler **302,** an outer body **304,** a sealing member **306,** an adhesive member **308** (e.g., KAPTON^{®} tape), a flow sensor **310** (e.g., a puff sensor or a pressure switch or sensor configured to detect a pressure drop or flow of air), a control component **312,** a spacer **314,** an electrical power source **316** (e.g., a battery, which may be rechargeable), a circuit board with an indicator **318** (e.g., a light emitting diode (LED)), a connector circuit **320,** and an end cap **322.** Examples of electrical power sources are described in U.S. Pat. App. Pub. No. 2010/0028766 to Peckerar et al.

The aerosol delivery device **100** may incorporate a sensor or detector for control of supply of electric power to a heat generation element when aerosol generation is desired (e.g., upon draw during use). As such, for example, there is provided a manner or method for turning off the power supply to the heat generation element when the aerosol generating piece is not being drawn upon during use, and for turning on the power supply to actuate or trigger the generation of heat by the heat generation element during draw.

For example, with respect to the flow sensor **310,** representative current regulating components and other current controlling components including various microcontrollers, sensors, and switches for aerosol delivery devices are described in U.S. Pat. Nos. 4,735,217 to Gerth et al.; 4,947,874 to Brooks et al.; 5,372,148 to McCafferty et al.; 6,040,560 to Fleischhauer et al.; 7,040,314 to Nguyen et al. 8,205,622 to Pan, and 8,881,737 to Collet et al.; U.S. Pat. Pub. Nos. 2009/0230117 to Fernando et al. and 2014/0270727 to Ampolini et al.; and U.S. Pat. App. Ser. No. 14/209,191, filed March 13, 2014, to Henry et al. Additional representative types of sensing or detection mechanisms, structures, components, configurations, and general methods of operation thereof, are described in U.S. Pat. Nos. 5,261,424 to Sprinkel, Jr.; 5,372,148 to McCafferty et al.; and PCT WO 2010/003480 to Flick.

In one example, the indicator **318** may include one or more light emitting diodes. The indicator can be in communication with the control component **312** through the connector circuit **320** and illuminate, for example, during a user drawing on a cartridge (e.g., the cartridge **102**) coupled to the coupler **302,** as detected by the flow sensor **310.** The end cap **322** may be adapted to make visible the illumination provided thereunder by the indicator. Accordingly, the indicator may illuminate during use of the aerosol delivery device **100** to simulate the lit end of a smoking article. However, in other examples, the indicator can be provided in varying numbers and can take on different shapes and can even be an opening in the outer body (such as for release of sound when such indicators are present).

Various elements that may be included in the control body are described in U.S. App. Ser. No. 14/193,961 to Worm et al., filed February 28, 2014. Still further components can be utilized in the aerosol delivery device of the present disclosure. For example, U.S. Pat. No. 5,154,192 to Sprinkel et al. discloses indicators for smoking articles; U.S. Pat. No. 5,261,424 to Sprinkel, Jr. discloses piezoelectric sensors that can be associated with the mouth-end of a device to detect user lip activity associated with taking a draw and then trigger heating; U.S. Pat. No. 5,372,148 to McCafferty et al. discloses a puff sensor for controlling energy flow into a heating load array in response to a pressure drop through a mouthpiece; U.S. Pat. No. 5,967,148 to Harris et al. discloses receptacles in a smoking device that include an identifier that detects a non-uniformity in infrared transmissivity of an inserted component and a controller that executes a detection routine as the component is inserted into the receptacle; U.S. Pat. No. 6,040,560 to Fleischhauer et al. describes a defined executable power cycle with multiple differential phases; U.S. Pat. No. 5,934,289 to Watkins et al. discloses photonic-optronic components; U.S. Pat. No. 5,954,979 to Counts et al. discloses means for altering draw resistance through a smoking device; U.S. Pat. No. 6,803,545 to Blake et al. discloses specific battery configurations for use in smoking devices; U.S. Pat. No. 7,293,565 to Griffen et al. discloses various charging systems for use with smoking devices; U.S. Pat. No. 8,402,976 to Fernando et al. discloses computer interfacing means for smoking devices to facilitate charging and allow computer control of the device; U.S. Pat. No. 8,689,804 to Fernando et al. discloses identification systems for smoking devices; and WO 2010/003480 to Flick discloses a fluid flow sensing system indicative of a puff in an aerosol generating system. Further examples of components related to electronic aerosol delivery articles and disclosing materials or components that may be used in the present article include U.S. Pat. No. 4,735,217 to Gerth et al.; U.S. Pat. No. 5,249,586 to Morgan et al.; U.S. Pat. No. 5,666,977 to Higgins et al.; U.S. Pat. No. 6,053,176 to Adams et al.; U.S. 6,164,287 to White; U.S. Pat No. 6,196,218 to Voges; U.S. Pat. No. 6,810,883 to Felter et al.; U.S. Pat. No. 6,854,461 to Nichols; U.S. Pat. No. 7,832,410 to Hon; U.S. Pat. No. 7,513,253 to Kobayashi; U.S. Pat. No. 7,896,006 to Hamano; U.S. Pat. No. 6,772,756 to Shayan; U.S. Pat. No. 8,156,944 and 8,375,957 to Hon; U.S. Pat. No. 8,794,231 to Thorens et al.; U.S. Pat. No. 8,851,083 to Oglesby et al.; U.S. Pat. Nos. 8,915,254 and 8,925,555 to Monsees et al.; U.S. Pat. App. Pub. Nos. 2006/0196518 and 2009/0188490 to Hon; U.S. Pat. App. Pub. No. 2010/0024834 to Oglesby et al.; U.S. Pat. App. Pub. No. 2010/0307518 to Wang; U.S. Pat. App. Pub. No. 2014/0261408 to DePiano et al.; WO 2010/091593 to Hon; and WO 2013/089551 to Foo.

Referring to FIGS. 1 and 2, during use, a user may draw on the mouthpiece **216** of the cartridge **102** of the aerosol delivery device **100.** This may pull air through an opening in the control body **104** or in the cartridge. For example, in one example, an opening may be defined between the coupler **302** and the outer body **304** of the control body, as described in U.S. Pat. App. Pub. No. 2014/0261408 to DePiano et al. However, the flow of air may be received through other parts of the aerosol delivery device in other example implementations. As noted above, in some examples, the cartridge may include the flow director **208.** The flow director may be configured to direct the flow of air received from the control body **104** to the heating element **224** of the atomizer **210.**

A sensor in the aerosol delivery device **100** (e.g., the flow sensor **310** in the control body **104**) may sense the puff. When the puff is sensed, the control body **104** may direct current to the heating element **224** through a circuit including the first heating terminal **220a** and the second heating terminal **220b.** Accordingly, the heating element may vaporize the aerosol precursor composition directed to an aerosolization zone from the reservoir substrate **212** by the liquid transport element **222.** Thus, the mouthpiece **216** may allow passage of air and entrained vapor (i.e., the components of the aerosol precursor composition in an inhalable form) from the cartridge **102** to a consumer drawing thereon.

As noted above, in some examples, the control body **104** may include the indicator **318** (e.g., an LED), which may be configured to illuminate an end of the control body. For example, the indicator may illuminate the end cap **322** during use of the aerosol delivery device **100** to simulate the lit end of a smoking article. However, it may be desirable to illuminate other or additional portions of an aerosol delivery device. Further, it may be desirable to transmit light within the aerosol delivery device to one or more locations positioned distally from the light source such that the position of the light source may be selected to facilitate assembly of the control body and/or provide other advantages.

Again, the emissive or electronic paper display **106** of example implementations of the present disclosure may be controllable to present information of the aerosol delivery device 100, whereas the implementations with electronic paper displays are not part of the claimed invention. This information may indicate, for example, an amount of aerosol precursor composition contained in or consumed from the cartridge **102** (e.g., reservoir substrate **212**), or an amount of charge contained in or consumed from the power source **316.** In some examples, this information may be presented in the form of icons.

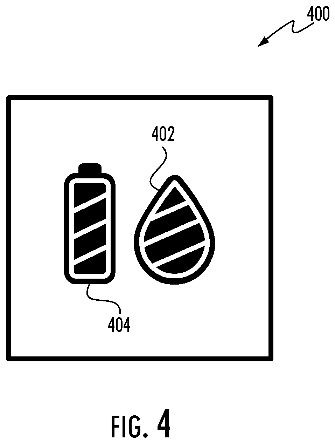

FIG. 4 illustrates information that may be presented by the emissive or electronic paper display 106, according to an example implementation of the present disclosure. As shown, the emissive or electronic paper display may present icons **402, 404** that represent respectively the aerosol precursor composition and power source **316.** The icon for the aerosol precursor composition may indicate the amount thereof contained in or consumed from the cartridge **102** (e.g., reservoir substrate **212**). Similarly, the icon for the power source may indicate an amount of charge contained in or consumed from the power source. As shown, each icon may be divided into a number of segments that represent incremental percentages (e.g., 10-20 %) of its respective consumable. The icon for the aerosol precursor composition may be divided into segments that represent an incremental amount of remaining aerosol precursor composition (contained in the cartridge). And the icon for the power source may be divided into segments that represent an incremental amount of remaining charge (contained in the power source).

The foregoing description of use of the article(s) can be applied to the various example implementations described herein through minor modifications, which can be apparent to the person of skill in the art in light of the further disclosure provided herein. The above description of use, however, is not intended to limit the use of the article but is provided to comply with all necessary requirements of disclosure of the present disclosure. Any of the elements shown in the article(s) illustrated in FIGS. 1-4 or as otherwise described above may be included in an aerosol delivery device according to the present disclosure.

Many modifications and other implementations of the disclosure set forth herein will come to mind to one skilled in the art to which this disclosure pertains having the benefit of the teachings presented in the foregoing descriptions and the associated drawings. Therefore, it is to be understood that the disclosure is not to be limited to the specific implementations disclosed, and that modifications and other implementations are intended to be included within the scope of the appended claims. Moreover, although the foregoing descriptions and the associated drawings describe example implementations in the context of certain example combinations of elements and/or functions, it should be appreciated that different combinations of elements and/or functions may be provided by alternative implementations without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An apparatus comprising:
at least one housing equipped with a heating element (224) and containing an aerosol precursor composition, the heating element (224) being configured to activate and vaporize components of the aerosol precursor composition; and
a flexible, emissive display (106) received on a surface of the at least one housing, and controllable to present information of an aerosol delivery device (100),
wherein the flexible, emissive display (106) is flexible so as to be moldable to a form factor of the surface of the at least one housing, the surface being non-planar, and
wherein the apparatus is an aerosol delivery device (100) or a cartridge (102) for an aerosol delivery device (100).

2. The apparatus of Claim 1, wherein the non-planar surface is substantially tubular in shape.

3. The apparatus of any one of Claim 1 or 2, wherein the emissive display (106) is controllable to present information that indicates an amount of aerosol precursor composition contained in or consumed from the at least one housing.

4. The apparatus of Claim 3, wherein the emissive display (106) being controllable to present information includes being controllable to present an icon that represents the aerosol precursor composition and indicates the amount thereof contained in or consumed from the at least one housing.

5. The apparatus of any one of Claims 1 to 4, wherein the heating element (224) is connected or connectable with a power source (316), and the emissive display (106) is controllable to present information that indicates an amount of charge contained in or consumed from the power source (316).

6. The apparatus of Claim 5, wherein the emissive display (106) being controllable to present information includes being controllable to present an icon that represents the power source (316) and indicates the amount of charge contained in or consumed therefrom.

7. The apparatus of Claim 5 or 6, wherein the emissive display (106) being controllable to present information includes being controllable to present information that also indicates an amount of aerosol precursor composition contained in or consumed from the at least one housing.

8. The apparatus of Claim 7, wherein the emissive display (106) being controllable to present information includes being controllable to present an icon that represents the power source (316) and indicates the amount of charge contained in or consumed therefrom, and an icon that represents the aerosol precursor composition and indicates the amount thereof contained in or consumed from the at least one housing.

9. A control body (104) coupled or coupleable with a cartridge (102) that is equipped with a heating element (224) and contains an aerosol precursor composition, the control body (104) being coupled or coupleable with the cartridge (102) to form an aerosol delivery device (100) in which the heating element (224) is configured to activate and vaporize components of the aerosol precursor composition, the control body (104) comprising:
at least one housing;
a flexible, emissive display (106) received on a surface of the at least one housing; and
a control component (206) contained within the at least one housing and configured to operate in an active mode in which the control body (104) is coupled with the cartridge (102), the control component (206) in the active mode being configured to control the heating element (224) to activate and vaporize components of the aerosol precursor composition, the control component (206) being further configured to control the emissive display (106) to present information of the cartridge (102), control body (104) or aerosol delivery device (100),
wherein the flexible, emissive display (106) is flexible so as to be moldable to a form factor of the surface of the at least one housing, the surface being non-planar.

10. The control body (104) of Claim 9, wherein the non-planar surface is substantially tubular in shape.

11. The control body (104) of any one of Claim 9 or 10, wherein the control component (206) is configured to control the emissive display (106) to present information that indicates an amount of aerosol precursor composition contained in or consumed from the at least one housing.

12. The control body (104) of Claim 11, wherein the control component (206) being configured to control the emissive display (106) includes being configured to control the emissive display (106) to present an icon that represents the aerosol precursor composition and indicates the amount thereof contained in or consumed from the at least one housing.

13. The control body (104) of any one of Claims 9 to 12, wherein the heating element (224) is connected or connectable with a power source (316), and the control component (206) is configured to control the emissive display (106) to present information that indicates an amount of charge contained in or consumed from the power source (316).

14. The control body (104) of Claim 13, wherein the control component (206) being configured to control the emissive display (106) includes being configured to control the emissive display (106) to present an icon that represents the power source (316) and indicates the amount of charge contained in or consumed therefrom.

15. The control body (104) of Claim 13 or 14, wherein the control component (206) being configured to control the emissive display (106) includes being configured to control the emissive display (106) to present information that also indicates an amount of aerosol precursor composition contained in or consumed from the at least one housing.

16. The control body (104) of Claim 15, wherein the control component (206) being configured to control the emissive display (106) includes being configured to control the emissive display (106) to present an icon that represents the power source (316) and indicates the amount of charge contained in or consumed therefrom, and an icon that represents the aerosol precursor composition and indicates the amount thereof contained in or consumed from the at least one housing.

## Patentansprüche

1. Eine Vorrichtung, umfassend:
mindestens ein Gehäuse, welches mit einem Heizelement (224) ausgestattet ist und eine Aerosol-Precursor-Zusammensetzung enthält, wobei das Heizelement (224) dazu ausgebildet ist, Komponenten der Aerosol-Precursor-Zusammensetzung zu aktivieren und zu verdampfen; und
eine flexible, emissive Anzeige (106), welche an einer Oberfläche des mindestens einen Gehäuses aufgenommen ist und kontrollierbar ist, um Informationen in Bezug auf eine Aerosolabgabevorrichtung (100) zu präsentieren,
wobei die flexible, emissive Anzeige (106) flexibel ist, um an einen Formfaktor der Oberfläche des mindestens einen Gehäuses anformbar zu sein, wobei die Oberfläche eine nicht-planare Oberfläche ist, und
wobei die Vorrichtung eine Aerosolabgabevorrichtung (100) oder eine Patrone (102) für eine Aerosolabgabevorrichtung (100) ist.

2. Die Vorrichtung nach Anspruch 1, wobei die nicht-planare Oberfläche eine im Wesentlichen rohrförmige Gestalt aufweist.

3. Die Vorrichtung nach einem der Ansprüche 1 oder 2, wobei die emissive Anzeige (106) kontrollierbar ist, um Informationen zu präsentieren, welche eine Menge an Aerosol-Precursor-Zusammensetzung angeben, welche in dem mindestens einen Gehäuse enthalten oder daraus verbraucht ist.

4. Die Vorrichtung nach Anspruch 3, wobei die emissive Anzeige (106), welche kontrollierbar ist, um Informationen zu präsentieren, umfasst, kontrollierbar zu sein, um ein Icon zu präsentieren, welches die Aerosol-Precursor-Zusammensetzung repräsentiert und die Menge derselben anzeigt, welche in dem mindestens einen Gehäuse enthalten oder daraus verbraucht ist.

5. Die Vorrichtung nach einem der Ansprüche 1 bis 4, wobei das Heizelement (224) mit einer Leistungsquelle (316) verbunden oder verbindbar ist und die emissive Anzeige (106) kontrollierbar ist, um Informationen zu präsentieren, welche eine in der Leistungsquelle (316) enthaltene oder aus derselben verbrauchte Ladungsmenge angeben.

6. Die Vorrichtung nach Anspruch 5, wobei die emissive Anzeige (106), welche kontrollierbar ist, um Informationen zu präsentieren, umfasst, kontrollierbar zu sein, um ein Icon zu präsentieren, welches die Leistungsquelle (316) repräsentiert und die in dieser enthaltene oder aus dieser verbrauchte Ladungsmenge angibt.

7. Die Vorrichtung nach Anspruch 5 oder 6, wobei die emissive Anzeige (106), welche kontrollierbar ist, um Informationen zu präsentieren, umfasst, kontrollierbar zu sein, um Informationen zu präsentieren, welche auch eine Menge an Aerosol-Precursor-Zusammensetzung angeben, die in dem mindestens einen Gehäuse enthalten oder aus demselben verbraucht ist.

8. Die Vorrichtung nach Anspruch 7, wobei die emissive Anzeige (106), welche kontrollierbar ist, um Informationen zu präsentieren, umfasst, kontrollierbar zu sein, um ein Icon zu präsentieren, welches die Leistungsquelle (316) repräsentiert und die in dieser enthaltene oder aus dieser verbrauchte Ladungsmenge angibt, und um ein Icon zu präsentieren, welches die Aerosol-Precursor-Zusammensetzung repräsentiert und die Menge derselben angibt, welche in dem mindestens einen Gehäuse enthalten oder aus demselben verbraucht ist.

9. Ein Kontrollkörper (104), welcher mit einer Patrone (102) gekoppelt oder koppelbar ist, welche mit einem Heizelement (224) ausgestattet ist und eine Aerosol-Precursor-Zusammensetzung enthält, wobei der Kontrollkörper (104) mit der Patrone (102) gekoppelt oder koppelbar ist, um eine Aerosolabgabevorrichtung (100) zu bilden, bei welcher das Heizelement (224) dazu ausgebildet ist, Komponenten der Aerosol-Precursor-Zusammensetzung zu aktivieren und zu verdampfen, wobei der Kontrollkörper (102) umfasst:
mindestens ein Gehäuse;
eine flexible, emissive Anzeige (106), welche an einer Oberfläche des mindestens einen Gehäuses aufgenommen ist; und
eine Kontrollkomponente (206), welche innerhalb des mindestens einen Gehäuses enthalten ist und dazu ausgebildet ist, in einem Aktivmodus zu arbeiten, in dem der Kontrollkörper (104) mit der Patrone (102) gekoppelt ist, wobei die Kontrollkomponente (206) in dem Aktivmodus dazu ausgebildet ist, das Heizelement (224) zu kontrollieren, um Komponenten der Aerosol-Precursor-Zusammensetzung zu aktivieren und zu verdampfen, wobei die Kontrollkomponente (206) ferner dazu ausgebildet ist, die emissive Anzeige (106) zu kontrollieren, um Informationen in Bezug auf die Patrone (102), den Kontrollkörper (104) oder die Aerosolabgabevorrichtung (100) zu präsentieren,
wobei die flexible, emissive Anzeige (106) flexibel ist, um an einen Formfaktor der Oberfläche des mindestens einen Gehäuses anformbar zu sein, wobei die Oberfläche eine nicht-planare Oberfläche ist.

10. Der Kontrollkörper (104) nach Anspruch 9, wobei die nicht-planare Oberfläche eine im Wesentlichen rohrförmige Gestalt aufweist.

11. Der Kontrollkörper (104) nach einem der Ansprüche 9 oder 10, wobei die Kontrollkomponente (206) dazu ausgebildet ist, die emissive Anzeige (106) zu kontrollieren, um Informationen zu präsentieren, welche eine Menge an Aerosol-Precursor-Zusammensetzung angeben, welche in dem mindestens einen Gehäuse enthalten oder daraus verbraucht ist.

12. Der Kontrollkörper (104) nach Anspruch 11, wobei die Kontrollkomponente (206), welche dazu ausgebildet ist, die emissive Anzeige (106) zu kontrollieren, umfasst, dazu ausgebildet zu sein, die emissive Anzeige (106) zu kontrollieren, um ein Icon zu präsentieren, welches die Aerosol-Precursor-Zusammensetzung repräsentiert und die Menge derselben angibt, welche in dem mindestens einen Gehäuse enthalten oder daraus verbraucht ist.

13. Der Kontrollkörper (104) nach einem der Ansprüche 9 bis 12, wobei das Heizelement (224) mit einer Leistungsquelle (316) verbunden oder verbindbar ist und die Kontrollkomponente (204) dazu ausgebildet ist, die emissive Anzeige (106) zu kontrollieren, um Informationen zu präsentieren, welche eine in der Leistungsquelle (316) enthaltene oder aus derselben verbrauchte Ladungsmenge angeben.

14. Der Kontrollkörper (104) nach Anspruch 13, wobei die Kontrollkomponente (206), welche dazu ausgebildet ist, die emissive Anzeige (106) zu kontrollieren, umfasst, dazu ausgebildet zu sein, die emissive Anzeige (106) zu kontrollieren, um ein Icon zu präsentieren, welches die Leistungsquelle (316) repräsentiert und die in dieser enthaltene oder aus dieser verbrauchte Ladungsmenge angibt.

15. Der Kontrollkörper (104) nach Anspruch 13 oder 14, wobei die Kontrollkomponente (206), welche dazu ausgebildet ist, die emissive Anzeige (106) zu kontrollieren, umfasst, dazu ausgebildet zu sein, die emissive Anzeige (106) zu kontrollieren, um Informationen zu präsentieren, welche auch eine Menge an Aerosol-Precursor-Zusammensetzung angeben, die in dem mindestens einen Gehäuse enthalten oder aus demselben verbraucht ist.

16. Der Kontrollkörper (104) nach Anspruch 15, wobei die Kontrollkomponente (206), welche dazu ausgebildet ist, die emissive Anzeige (106) zu kontrollieren, umfasst, dazu ausgebildet zu sein, die emissive Anzeige (106) zu kontrollieren, um ein Icon zu präsentieren, welches die Leistungsquelle (316) repräsentiert und die in dieser enthaltene oder aus dieser verbrauchte Ladungsmenge angibt, und um ein Icon zu präsentieren, welches die Aerosol-Precursor-Zusammensetzung repräsentiert und die Menge derselben angibt, welche in dem mindestens einen Gehäuse enthalten oder aus demselben verbraucht ist.

## Revendications

1. Appareil comprenant :
au moins un boîtier équipé d'un élément chauffant (224) et contenant une composition de précurseur d'aérosol, l'élément chauffant (224) étant configuré pour activer et vaporiser des composants de la composition de précurseur d'aérosol ; et
un dispositif d'affichage flexible émissif (106) reçu sur une surface de l'au moins un boîtier, et commandable pour présenter des informations d'un dispositif de fourniture d'aérosol (100),
dans lequel le dispositif d'affichage flexible émissif (106) est flexible de manière à être déformable selon un facteur de forme de la surface de l'au moins un boîtier, la surface étant non plane, et
dans lequel l'appareil est un dispositif de fourniture d'aérosol (100) ou une cartouche (102) pour un dispositif de fourniture d'aérosol (100).

2. Appareil selon la revendication 1, dans lequel la surface non plane est de forme sensiblement tubulaire.

3. Appareil selon l'une quelconque des revendications 1 ou 2, dans lequel le dispositif d'affichage émissif (106) est commandable pour présenter des informations qui indiquent une quantité de composition de précurseur d'aérosol contenue dans ou consommée à partir de l'au moins un boîtier.

4. Appareil selon la revendication 3, dans lequel le dispositif d'affichage émissif (106) commandable pour présenter des informations inclut le fait d'être commandable pour présenter une icône qui représente la composition de précurseur d'aérosol et indique la quantité de celle-ci contenue dans ou consommée à partir de l'au moins un boîtier.

5. Appareil selon l'une quelconque des revendications 1 à 4, dans lequel l'élément chauffant (224) est connecté ou connectable à une source d'alimentation (316), et le dispositif d'affichage émissif (106) est commandable pour présenter des informations qui indiquent une quantité de charge contenue dans ou consommée à partir de la source d'alimentation (316).

6. Appareil selon la revendication 5, dans lequel le dispositif d'affichage émissif (106) commandable pour présenter des informations inclut le fait d'être commandable pour présenter une icône qui représente la source d'alimentation (316) et indique la quantité de charge contenue dans ou consommée à partir de celle-ci.

7. Appareil selon la revendication 5 ou 6, dans lequel le dispositif d'affichage émissif (106) commandable pour présenter des informations inclut le fait d'être commandable pour présenter des informations qui indiquent également une quantité de composition de précurseur d'aérosol contenue dans ou consommée à partir de l'au moins un boîtier.

8. Appareil selon la revendication 7, dans lequel le dispositif d'affichage émissif (106) commandable pour présenter des informations inclut le fait d'être commandable pour présenter une icône qui représente la source d'alimentation (316) et indique la quantité de charge contenue dans ou consommée à partir de celle-ci, et une icône qui représente la composition de précurseur d'aérosol et indique la quantité de celle-ci contenue dans ou consommée à partir de l'au moins un boîtier.

9. Corps de commande (104) couplé ou couplable à une cartouche (102) qui est équipée d'un élément chauffant (224) et contient une composition de précurseur d'aérosol, le corps de commande (104) étant couplé ou couplable à la cartouche (102) pour former un dispositif de fourniture d'aérosol (100) dans lequel l'élément chauffant (224) est configuré pour activer et vaporiser des composants de la composition de précurseur d'aérosol, le corps de commande (104) comprenant :
au moins un boîtier ;
un dispositif d'affichage flexible émissif (106) reçu sur une surface de l'au moins un boîtier ; et
un composant de commande (206) contenu au sein de l'au moins un boîtier et configuré pour fonctionner dans un mode actif dans lequel le corps de commande (104) est couplé à la cartouche (102), le composant de commande (206) dans le mode actif étant configuré pour commander l'élément chauffant (224) pour activer et vaporiser des composants de la composition de précurseur d'aérosol, le composant de commande (206) étant en outre configuré pour commander le dispositif d'affichage émissif (106) pour présenter des informations de la cartouche (102), du corps de commande (104) ou du dispositif de fourniture d'aérosol (100),
dans lequel le dispositif d'affichage flexible émissif (106) est flexible de manière à être déformable selon un facteur de forme de la surface de l'au moins un boîtier, la surface étant non plane.

10. Corps de commande (104) selon la revendication 9, dans lequel la surface non plane est de forme sensiblement tubulaire.

11. Corps de commande (104) selon l'une quelconque des revendications 9 ou 10, dans lequel le composant de commande (206) est configuré pour commander le dispositif d'affichage émissif (106) pour présenter des informations qui indiquent une quantité de composition de précurseur d'aérosol contenue dans ou consommée à partir de l'au moins un boîtier.

12. Corps de commande (104) selon la revendication 11, dans lequel le composant de commande (206) configuré pour commander le dispositif d'affichage émissif (106) inclut le fait d'être configuré pour commander le dispositif d'affichage émissif (106) pour présenter une icône qui représente la composition de précurseur d'aérosol et indique la quantité de celle-ci contenue dans ou consommée à partir de l'au moins un boîtier.

13. Corps de commande (104) selon l'une quelconque des revendications 9 à 12, dans lequel l'élément chauffant (224) est connecté ou connectable à une source d'alimentation (316), et le composant de commande (206) est configuré pour commander le dispositif d'affichage émissif (106) pour présenter des informations qui indiquent une quantité de charge contenue dans ou consommée à partir de la source d'alimentation (316).

14. Corps de commande (104) selon la revendication 13, dans lequel le composant de commande (206) configuré pour commander le dispositif d'affichage émissif (106) inclut le fait d'être configuré pour commander le dispositif d'affichage émissif (106) pour présenter une icône qui représente la source d'alimentation (316) et indique la quantité de charge contenue dans ou consommée à partir de celle-ci.

15. Corps de commande (104) selon la revendication 13 ou 14, dans lequel le composant de commande (206) configuré pour commander le dispositif d'affichage émissif (106) inclut le fait d'être configuré pour commander le dispositif d'affichage émissif (106) pour présenter des informations qui indiquent également une quantité de composition de précurseur d'aérosol contenue dans ou consommée à partir de l'au moins un boîtier.

16. Corps de commande (104) selon la revendication 15, dans lequel le composant de commande (206) configuré pour commander le dispositif d'affichage émissif (106) inclut le fait d'être configuré pour commander le dispositif d'affichage émissif (106) pour présenter une icône qui représente la source d'alimentation (316) et indique la quantité de charge contenue dans ou consommée à partir de celle-ci, et une icône qui représente la composition de précurseur d'aérosol et indique la quantité de celle-ci contenue dans ou consommée à partir de l'au moins un boîtier.
